# FASCICULE DE BREVET EUROPEEN

(11) **EP 1 005 851 B1**
(45) Date de publication et mention de la délivrance du brevet: **03.07.2002**
(21) Numéro de dépôt: 99403002.1
(22) Date de dépôt: 02.12.1999
(51) Int. Cl.: A61K 7/00

(54) **Composition cosmétique ou dermatologique comprenant au moins un alkynyl carbamate et au moins un polyol**
Kosmetische oder dermatologische Zusammensetzung enthaltend mindestens ein Alkynylcarbamat und mindestens ein Polyol
Cosmetic or dermatological composition comprising at least one alkynyl carbamate and at least one polyol

(30) Priorité: 03.12.1998 FR 9815304
(43) Date de publication de la demande: 07.06.2000
(73) Titulaire: L'OREAL, 75008 Paris (FR)
(72) Inventeur: Saint-Leger, Didier, 92400 Courbevoie (FR)
(74) Mandataire: Casalonga, Axel

(56) Documents cités:
- EP-A- 0 705 595
- WO-A-95/29588
- WO-A-96/24329
- WO-A-99/49730
- FR-A- 2 751 217
- FR-A- 2 758 722

## Description

La présente invention est relative à des compositions cosmétiques ou dermatologiques, comprenant au moins un alkynyl carbamate et au moins un polyol; à l'utilisation de ces compositions en cosmétique; et à l'utilisation pour la préparation d'une composition cosmétique ou dermatologique d'un alkynyl carbamate et d'un polyol pour le traitement des désordres cutanés inflammatoires et/ou desquamatifs liés à Malassezia spp.

Les désordres desquamatifs du cuir chevelu tels que les pellicules ou les dermites séborrhiques sont liés à la présence d'une levure caractéristique appelée le Malassezia ovalis, levure anciennement dénommée Pityrosporum (P. ovale et P. orbiculare).

Par ailleurs, il est connu que cette levure est capable de changer de forme et de métabolisme. En particulier, elle peut évoluer vers une forme filamenteuse (Malassezia furfur) responsable du désordre inflammatoire et pigmentaire appelé Pityriasis versicolor.

La demande de brevet WO 95/29588 (ISP CHEMICAL INC.) décrit des compositions contenant des mélanges de composés biocides ayant une activité conservatrice pour différents produits commerciaux. Ces compositions contiennent (a) un ou plusieurs composés de type méthylol ou leurs équivalents et (b) l'alcool iodopropynylique, ou ses esters, carbamates ou éthers dans un rapport pondéral (a) : (b) compris entre 100:1 et 2000:1.

La demande de brevet WO 96/24329 (PROCTER & GAMBLE COMPA-NY) décrit des compositions de soin individuel pour le nettoyage de la zone périnéale et la protection contre la dermatite périnéale qui contiennent de l'eau, de la diméthicone, un émulsifiant polymèrique et éventuellement un polyol hydrosoluble, un agent anti-microbien, et un agent chélatant.

La demande de brevet FR 2758722 (PACIFIC CORPORATION) décrit des compositions cosmétiques de soins capillaires ayant une action anti-bactérienne vis-à-vis des micro-organismes responsables des pellicules. Ces compositions contiennent du butylcarbamate d'iodopropynyle et/ou une solution de pyrithione de zinc solubilisée par un agent chélatant tel que le triacétate de N-acyl-éthylènediamine.

La demande de brevet FR-A-2751 217 (L'ORÉAL) décrit l'utilisation de l'iodo-3 propynyl-2 butylcarbamate dans une composition cosmétique ou dermatologique, comme agent actif contre certains micro-organismes pour traiter les dermatoses associées à la séborrhée telles que l'acné et les pellicules.

Le traitement usuel de toutes ces affections fait intervenir l'utilisation d'agents antifongiques dans un milieu, tel qu'un shampooing, un gel ou une lotion, qui est apte à répartir ces agents et à les déposer sur les téguments.

Le pouvoir antifongique de ces agents est limité; de plus, la rémanence du pouvoir antifongique est faible. Ainsi, les traitements utilisant ces antifongiques sont d'efficacité très moyenne, voir faible.

La demanderesse a donc cherché à résoudre ces problèmes et à obtenir des traitements plus efficaces.

Elle a découvert, de façon surprenante, qu'en associant un alkynyl carbamate avec certains polyols, il était possible d'obtenir des traitements bien plus efficaces que ceux de l'art antérieur. En effet, la demanderesse a découvert de façon surprenante que ces associations présentaient une forte et durable activité antifongique dirigée contre Malassezia spp.

Il a notamment été remarqué que les associations de ces composés, à des doses où chacune de leur activité propre est perdue, restent efficaces et présentent une activité antifongique renforcée.

Il a également été constaté que les associations de l'invention présentaient une bonne solubilité et stabilité dans les milieux habituellement utilisés pour l'application de compositions antifongiques, ainsi qu'une bonne tolérance cutanée.

La présente invention concerne donc une composition cosmétique ou dermatologique, comprenant dans un milieu physiologiquement acceptable, au moins un alkynyl carbamate et au moins un polyol.

Un autre objet de l'invention concerne l'utilisation de ces compositions comme composition cosmétique antifongique ou antipelliculaire.

Un objet de l'invention et aussi l'utilisation d'un alkynyl carbamate et d'un polyol pour la préparation d'une composition dermatologique antipelliculaire ou antifongique.

Un autre objet de l'invention consiste en un procédé de traitement cosmétique pour l'élimination de pellicules des cheveux et du cuir chevelu utilisant ces compositions.

D'autres objets de l'invention apparaîtront à la lumière de la description et des exemples qui suivent.

La présente invention a essentiellement pour objet une composition cosmétique ou dermatologique caractérisée par le fait qu'elle comprend dans un milieu physiologiquement acceptable :
- au moins un alkynyl carbamate de formule (I) suivante : 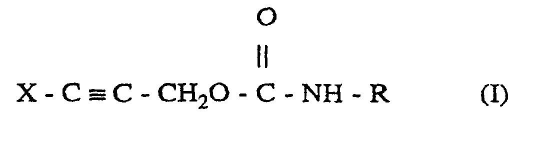 dans laquelle :
   X désigne un atome d'halogène
   R désigne un atome d'hydrogène,
      un groupement alkyl,
      un groupement hydroxyalkyl,
- et au moins un polyol choisi parmi les polyols non étherifiés, les (poly)alkyl C₁-C₁₀ ou (poly)alkényl C₂-C₂₀ éthers de polyol et les glycosyl éthers de polyol; le polyol étant saturé ou non, linéaire, ramifié ou cyclique, porteur ou non d'un hétéroatome, porteur ou non de fonctions chimiques complémentaires choisies parmi les fonctions carbonyle et carboxyle.

Dans le cadre de la présente invention :

Un atome d'halogène désigne un atome de fluor, de chlore, de bromure ou d'iode et préférentiellement un atome de d'iode.

Les groupements alkyle désignent des groupements de 1 à 20 atomes de carbone linéaires ou ramifiés comme par exemple des groupements octyle, nonyle, décyle, dodécyle ou pentadécyle. Les groupements alkyle désignent préférentiellement des groupements alkyle inférieurs de 1 à 4 atomes de carbone à chaîne linéaire ou ramifiée comme par exemple le groupement méthyle, éthyle, propyle, isopropyle, n-propyle, butyle, n-butyle ou tert.-butyle.

Les compositions de l'invention contiennent préférentiellement des composés de formule (I) dans laquelle X désigne un atome d'iode et R désigne un groupement alkyle inférieur de 1 à 4 atomes de carbone.

Encore plus préférentiellement, elles contiennent au moins du 3-iodo-2-propynyl-butyl carbamate.

Selon l'invention, les polyols représentent des composés saturés ou non, linéaires, ramifiés ou cycliques, porteur ou non d'un hétéroatome, porteur ou non de fonctions chimiques complémentaires choisies parmi les fonctions carbonyle et carboxyle.

Parmi ces composés, on peut citer l'érythritol, l'arabitol, l'adonitol, le sorbitol, le dulcitol, le 1,2 butanediol, le 1,3 butanediol, le 1,4 butanediol, le 2,3 butanediol, le 1,5 pentanediol, le 2,4 pentanediol, le 2 méthyl 2,4 pentanediol, le 1,6 hexanediol, le 2,3 diméthyl 2,3 butanediol, le 2,2 diéthyl 1,3 propanediol, le 2 éthyl 1,3 hexanediol, le 2,2,4 triméthyl 1,3 pentanediol, le 2 éthyl 2 butyl 1,3 propanediol, le 1,2,4 butanetriol, le 1,2,6-hexanetriol, le 2,2 dihydroxyméthyl 1 butanol, le tetraméthylolméthane (pentaérythritol), l'α-monométhyléther de glycérine, l'α-mono-n butyléther de glycérine et l'acide 2-O-α-D-glucopyranosyl-L ascorbique.

Le polyol préférentiellement utilisé est le 3-(Ethyl-2-hexyle oxy)-1,2 propanediol (C₁₁H₂₄O₃).

L'alkynyl carbamate et le polyol peuvent être simultanément présents dans des proportions individuelles comprises entre 0,001% et 10% en poids par rapport au poids total de la composition et préférentiellement dans des proportions comprises entre 0,01% et 5% en poids par rapport au poids total de la composition.

Le rapport pondéral de l'alkynyl carbamate et du polyol peut varier de 0,01 à 100 et préférentiellement de 0,05 à 5.

Les compositions de l'invention peuvent présenter un pH compris entre 2 et 12.

Le milieu physiologiquement acceptable est généralement constitué par de l'eau ou par un mélange d'eau et d'au moins un solvant organique acceptable d'un point de vue physiologique en vue d'une application topique. Parmi ces solvants, on peut mentionner les alcools inférieurs en C₁-C₄ tels que l'éthanol, l'alcool isopropylique, les alkylène glycol tel que le propylène glycol, l'éther monobutylique de l'éthylène glycol, les mono(ou di)éthyl(ou méthyl)éthers du propylèneglycol et du dipropylène glycol. Lorsqu'ils sont présents, ces solvants représentent de préférence de 1 à 80% en poids du poids total de la composition.

Les compositions selon l'invention peuvent se présenter sous forme de lotions, de shampooings, de mousses, de crèmes, de gels, de sticks, de sprays, de baumes, de poudres, de savons solides ou liquides.

Les compositions conformes à l'invention peuvent éventuellement contenir en outre divers additifs autres que ceux définis ci-avant, qui n'altèrent pas les propriétés des compositions de l'invention, tels que des agents tensio-actifs anioniques, amphotères ou zwittérioniques, non-ioniques, des agents de mises en suspension, des polymères anioniques, non-ioniques, cationiques ou amphotères, des protéines, des huiles, des cires, des résines et/ou des gommes de silicone, des agents acidifiants ou alcalinisants, des agents conservateurs, des parfums ou autres adjuvants couramment utilisés en cosmétique ou en dermatologie.

Les compositions selon l'invention peuvent également contenir d'autres agents anti-bactériens que ceux décrits précédemment tels que la chloramine T, l'Irgasan DP 300, la chloramine B, le 1,3-dibromo 5,5-diméthylhydantoïne, le 1,3-dichloro 5,5-diméthylhydantoïne, le 3-bromo 1-chloro 5,5-diméthylhydantoïne ou la N-chlorosuccinimide.

Elles peuvent aussi contenir des agents antifongiques comme par exemple le sulfure de Sélenium, le zinc pyridinethione ou l'octopyrox.

L'invention a ainsi pour objet l'utilisation d'une composition telle que définie ci-avant pour le traitement cosmétique des cheveux et du cuir chevelu.

L'invention a encore pour objet l'utilisation d'une composition telle que définie ci-dessus, comme composition cosmétique antifongique.

Plus particulièrement, les compositions de l'invention sont préférentiellement utilisées comme compositions cosmétiques antipelliculaires.

L'invention concerne aussi l'utilisation d'alkynyl carbamate comme défini ci-dessus et d'au moins un polyol tel que défini ci-dessus pour la préparation d'une composition dermatologique antifongique ou pour la préparation d'une composition dermatologique destinée au traitement des désordres cutanés inflammatoires et/ou desquamatifs liés à Malassezia spp et plus particulièrement au traitement antipelliculaire.

La présente invention a aussi pour objet un procédé de traitement cosmétique dans lequel les compositions cosmétiques selon l'invention sont utilisées pour l'élimination des pellicules des cheveux et du cuir chevelu et elles sont appliquées, dans ce cas-là, sur des cheveux humides ou secs; ces applications étant suivies éventuellement d'un rinçage.

Dans une forme de réalisation préférée de l'invention, les compositions selon l'invention sont utilisées comme shampooings pour le lavage et le traitement des cheveux et du cuir chevelu.

Ils sont appliqués, dans ce cas-là, sur des cheveux humides ou secs dans des quantités efficaces pour les laver, cette application étant suivie d'un rinçage.

Les exemples qui suivent illustrent la présente invention sans toutefois présenter un caractère limitatif.

Dans les exemples qui suivent, on désigne par:
Composé A: le 3-iodo-2-propynyl butylcarbamate, et par
Composé B: le 3-(éthyl-2-hexyloxy)-1,2propanediol

### Exemple I : Lotion anti-pelliculaire

| | |
|---|---|
| Composé A | 0,1 g MA |
| Composé B | 0,2 g |
| Ethanol 95° | 30 g |
| Eau | qsp 100 g |

Cette solution est appliquée quotidiennement à raison de 6 ml sur le cuir chevelu et ce pendant 1 à 2 semaines. On constate alors une amélioration notable de l'état pelliculaire.

### Exemple II : Shampooing anti-pelliculaire

| | |
|---|---|
| Composé A | 0,1 g MA |
| Composé B | 1,0 g |
| Lauryl éther (2,2 OE) sulfate de sodium | 14 g MA |
| Cocoylbétaine | 2,4 g MA |
| Eau | qsp 100 g |

Ce shampooing est utilisé quotidiennement à raison de 10 g par chevelure avec un temps de pose de l'ordre d'une minute et ceci pendant une période de 2 semaines. On observe alors une nette amélioration de l'état pelliculaire.

### Exemple III : Shampooing antipelliculaire

| | |
|---|---|
| Composé A | 0,1 g MA |
| Composé B | 1,0 g |
| Lauryléther (2,2 OE) sulfate de sodium | 8 g MA |
| Alkyl C₈/C₁₀/C₁₂/C₁₄ Polyglycoside (1,4) | 6 g MA |
| Eau | qsp 100 g |

Ce shampooing est utilisé quotidiennement à raison de 10 g par chevelure avec un temps de pose de l'ordre d'une minute et ceci pendant une période de 2 semaines. On observe alors une nette amélioration de l'état pelliculaire.

### Exemple IV: Détermination de l'activité antifongique de l'association des composés (A) et (B)

L'activité de l'association du composé (A) et du composé (B) a été déterminée par une méthode classique dite de CMI (Concentration Minimale Inhibitrice), la CMI correspondant à la concentration à laquelle un produit donné inhibe la pousse d'une souche donnée dans des conditions définies.
En l'occurrence, des souches de Pityrosporum ovale de l'Institut Pasteur référence CIP 1363.82 ont été utilisées.
Un inoculum d'une culture fraîche, de 1 à 10.10⁷ cellules/ml est introduit dans un milieu gélose de Sabouraud + Tween 40 à 10g/l + mono oléate de glycérol à 2.5 g/l, en présence ou non du composé ou de l'association des composés à étudier, et incubé 48 heures à 30°C.
La concentration du composé ou de l'association de composés à partir de laquelle la pousse du micro-organisme est complètement inhibée (absence de trouble du milieu) est ainsi déterminée.

Pour le composé (A) en tant que tel et le composé (B) en tant que tel, non associés entre eux, à la concentration de CMI/2, la pousse est identique à celle du milieu témoin, milieu sans composé antibactérien.
La pousse est en revanche bloquée par la combinaison composé (A) à CMI/2 + composé (B) à CMI/2.

Ces résultats montrent donc que cette association de composés, à des doses où chacune de leur activité propre est perdue, reste efficace et présente une activité antifongique renforcée.

## Revendications

1. Composition cosmétique ou dermatologique **caractérisée par le fait qu'**elle comprend dans un milieu physiologiquement acceptable :
• au moins un alkynyl carbamate de formule (I) suivante : 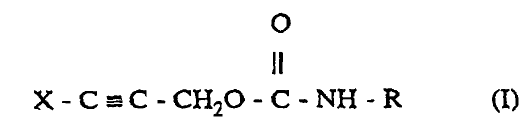 dans laquelle :
X désigne un atome d'halogène
R désigne un atome d'hydrogène,
un groupement alkyl,
un groupement hydroxyalkyl,
• et au moins un polyol choisi parmi l'érythritol, l'arabitol, l'adonitol, le sorbitol, le dulcitol, le 1,2 butanediol, le 1,3 butanediol, le 1,4 butanediol, le 2,3 butanediol, le 1,5 pentanediol, le 2,4 pentanediol, le 2 méthyl 2,4 pentanediol, le 1,6 hexanediol, le 2,3 diméthyl 2,3 butanediol, le 2,2 diéthyl 1,3 propanediol, le 2 éthyl 1,3 hexanediol, le 2,2,4 triméthyl 1,3 pentanediol, le 2 éthyl 2 butyl 1,3 propanediol, le 1,2,4 butanetriol, le 1,2,6-hexanetriol, le 2,2 dihydroxyméthyl 1 butanol, le tetraméthylolméthane, l'α-monométhyléther de glycérine, l'α-mono-n butyléther de glycérine, l'acide 2-O-α-D-glucopyranosyl-L ascorbique et le 3-(Ethyl-2-hexyle oxy)-1,2-propanediol,
• le rapport pondéral du composé (I) au polyol variant de 0,01 à 100.

2. Composition selon la revendication 1, **caractérisée en ce qu'**elle contient au moins un composé de formule (I) dans lequel X désigne un atome d'iode et R désigne un radical alkyl de 1 à 4 atomes de carbone.

3. Composition selon la revendication 1 ou 2, **caractérisée en ce que** le composé de formule (I) est le 3-iodo-2-propynyl-butyl carbamate.

4. Composition selon l'une quelconque des revendications 1 à 3, **caractérisée par le fait que** le composé de formule (I) et le polyol sont simultanément présents dans des proportions individuelles comprises entre 0,001% et 10% en poids par rapport au poids total de la composition.

5. Composition selon l'une quelconque des revendications 1 à 4, **caractérisée par le fait que** le composé de formule (I) et le polyol sont simultanément présents dans des proportions individuelles comprises entre 0,01% et 5% en poids par rapport au poids total de la composition.

6. Composition selon l'une quelconque des revendications 1 à 5, **caractérisée par le fait que** le rapport pondéral du composé (I) au polyol varie de 0,05 à 5.

7. Composition selon l'une quelconque des revendications 1 à 6, **caractérisée par le fait qu'**elle présente un pH compris entre 2 et 12.

8. Composition selon l'une quelconque des revendications 1 à 7, **caractérisée par le fait que** le milieu physiologiquement acceptable est constitué par de l'eau ou par un mélange d'eau et d'au moins un solvant organique choisi parmi les alcools inférieurs en C₁-C₄, les alkylène glycol, l'éther monobutylique de l'éthylène glycol, les mono(ou di)éthyl(ou méthyl)éthers du propylèneglycol et le dipropylène glycol présents dans des proportions comprises entre 1 et 80% en poids par rapport au poids total de la composition.

9. Composition selon l'une quelconque des revendications 1 à 8, **caractérisée par le fait qu'**elle se présente sous forme de lotions, de shampooings, de mousses, de crèmes, de gels, de sticks, de sprays, de baumes, de poudres, de savons solides ou liquides.

10. Composition selon l'une quelconque des revendications 1 à 9, **caractérisée par le fait que** la composition contient des additifs autres que les composés définis dans la revendication 1, choisis parmi des agents tensio-actifs anioniques, amphotères ou zwittérioniques, non-ioniques. des agents de mises en suspension, des polymères anioniques, non-ioniques, cationiques ou amphotères, des protéines, des huiles, des cires, des résines et/ou des gommes de silicone, des agents acidifiants ou alcalinisants, des agents conservateurs, des parfums, des antibactériens, des antifongiques ou autres adjuvants couramment utilisés en cosmétique ou en dermatologie.

11. Utilisation d'une composition selon l'une quelconque des revendications 1 à 10 pour le traitement cosmétique des cheveux et du cuir chevelu.

12. Utilisation non-thérapeutique d'une composition telle que définie dans l'une quelconque des revendications 1 à 10, comme composition cosmétique antifongique.

13. Utilisation non-thérapeutique d'une composition telle que définie dans l'une quelconque des revendications 1 à 10, comme composition cosmétique antipelliculaire.

14. Utilisation d'un alkynyl carbamate tel que défini dans l'une des revendications 1 à 3 et d'au moins un polyol tel que défini dans la revendication 1 pour la préparation d'une composition dermatologique destinée au traitement des désordres cutanés inflammatoires et/ou desquamatifs liés à Malassezia spp et plus particulièrement au traitement antipelliculaire.

15. Procédé de traitement cosmétique pour l'élimination des pellicules des cheveux et du cuir chevelu, **caractérisé par le fait que** l'on applique une composition cosmétique définie selon l'une quelconque des revendications 1 à 10 sur des cheveux humides ou secs, et que l'on fait suivre éventuellement d'un rinçage.

## Patentansprüche

1. Kosmetische oder dermatologische Zusammensetzung, **dadurch gekennzeichnet, daß** sie in einem physiologisch akzeptablen Medium enthält:
• mindestens ein Alkinylcarbamat der folgenden Formel (I): 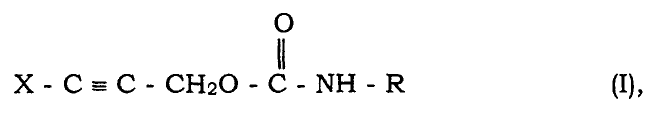 worin bedeuten:
X ein Halogenatom, und
R ein Wasserstoffatom, eine Alkylgruppe oder eine Hydroxyalkylgruppe, und
• mindestens ein Polyol, das ausgewählt ist unter: Erythrit, Arabit, Adonit, Sorbit, Dulcit, 1,2-Butandiol, 1,3-Butandiol, 1,4-Butandiol, 2,3-Butandiol, 1,5-Pentandiol, 2,4-Pentandiol, 2-Methyl-2,4-pentandiol, 1,6-Hexandiol, 2,3-Dimethyl-2,3-butandiol, 2,2-Diethyl-1,3-propandiol, 2-Ethyl-1,3-hexandiol, 2,2,4-Trimethyl-1,3-pentandiol, 2-Ethyl-2-butyl-1,3-propandiol, 1,2,4-Butantriol, 1,2,6-Hexantriol, 2,2-Dihydroxymethyl-1-butanol, Tetramethylolmethan, dem α-Mono-methylether von Glycerin, dem α-Mono-n-butylether von Glycerin, 2-O-α-D-Glucopyranosyl-L-ascorbinsäure und 3-(2-Ethylhexyloxy)-1,2-propandiol,
• wobei das Gewichtsverhältnis der Verbindung (I) und des Polyols im Bereich von 0,01 bis 100 liegt.

2. Zusammensetzung nach Anspruch 1, **dadurch gekennzeichnet, daß** sie mindestens eine Verbindung der Formel (I) enthält, worin X ein Iodatom bedeutet und R eine Alkylgruppe mit 1 bis 4 Kohlenstoffatomen ist.

3. Zusammensetzung nach Anspruch 1 oder 2, **dadurch gekennzeichnet, daß** die Verbindung der Formel (I) das 3-Iod-2-propinylbutylcarbamat ist.

4. Zusammensetzung nach einem der Ansprüche 1 bis 3, **dadurch gekennzeichnet, daß** die Verbindung der Formel (I) und das Polyol beide in Mengenanteilen von jeweils 0,001 bis 10 Gew.-%, bezogen auf das Gesamtgewicht der Zusammensetzung, vorliegen.

5. Zusammensetzung nach einem der Ansprüche 1 bis 4, **dadurch gekennzeichnet, daß** die Verbindung der Formel (I) und das Polyol beide jeweils in Mengenanteilen von 0,01 bis 5 Gew.-%, bezogen auf das Gesamtgewicht der Zusammensetzung, vorliegen.

6. Zusammensetzung nach einem der Ansprüche 1 bis 5, **dadurch gekennzeichnet, daß** das Gewichtsverhältnis der Verbindung (I) und des Polyols im Bereich von 0,05 bis 5 liegt.

7. Zusammensetzung nach einem der Ansprüche 1 bis 6, **dadurch gekennzeichnet, daß** sie einen pH-Wert von 2 bis 12 aufweist.

8. Zusammensetzung nach einem der Ansprüche 1 bis 7, **dadurch gekennzeichnet, daß** das physiologisch akzeptable Medium aus Wasser oder einem Gemisch von Wasser und mindestens einem organischen Lösungsmittel besteht, das unter den niederen C₁₋₄-Alkoholen, Alkylenglykolen, Ethylenglykolmonobutylether und den Mono(oder di)ethyl(oder methyl)ethern von Propylenglykol und Dipropylenglykol ausgewählt ist, wobei sie in Mengenanteilen von 1 bis 80 Gew.-%, bezogen auf das Gesamtgewicht der Zusammensetzung, vorliegen.

9. Zusammensetzung nach einem der Ansprüche 1 bis 8, **dadurch gekennzeichnet, daß** sie in Form von Lotionen, Haarwaschmitteln, Schäumen, Cremes, Gelen, Stiften, Sprays, Balsamen, Pulvern, festen Seifen oder flüssigen Seifen vorliegen.

10. Zusammensetzung nach einem der Ansprüche 1 bis 9, **dadurch gekennzeichnet, daß** die Zusammensetzung Zusatzstoffe enthält, die von den in Anspruch 1 definierten Verbindungen verschieden sind und die ausgewählt sind unter: anionischen, amphoteren oder zwitterionischen oder nichtionischen grenzflächenaktiven Stoffen, Suspendiermitteln, anionischen, nicht ionischen, kationischen oder amphoteren Polymeren, Proteinen, Siliconölen, Siliconwachsen, Siliconharzen und/oder Silicongummis, Alkalisieruns- oder Ansäuerungsmitteln, Konservierungsmitteln, Parfums, antibakteriellen Wirkstoffen, fungiziden Wirkstoffen und weiteren Zusatzstoffen, die häufig in der Kosmetik oder Dermatologie verwendet werden.

11. Verwendung einer Zusammensetzung nach einem der Ansprüche 1 bis 10 zur kosmetischen Behandlung der Haare und der Kopfhaut.

12. Nicht therapeutische Verwendung einer Zusammensetzung nach einem der Ansprüche 1 bis 10 als fungizide kosmetische Zusammensetzung.

13. Nicht therapeutische Verwendung einer Zusammensetzung nach einem der Ansprüche 1 bis 10 als kosmetische Zusammensetzung gegen Schuppen.

14. Verwendung eines Alkinylcarbamats nach einem der Ansprüche 1 bis 3 und mindestens eines Polyols nach Anspruch 1 zur Herstellung einer dermatologischen Zusammensetzung, die zur Behandlung von entzündlichen und/oder desquamativen Hautstörungen, die mit Malassezia spp. in Verbindung stehen, und insbesondere zur Behandlung von Schuppen vorgesehen ist.

15. Verfahren zur kosmetischen Behandlung, um Schuppen der Haare und der Kopfhaut zu beseitigen, **dadurch gekennzeichnet, daß** eine kosmetische Zusammensetzung nach einem der Ansprüche 1 bis 10 auf die feuchten oder trockenen Haare aufgetragen wird und anschließend gegebenenfalls gespült wird.

## Claims

1. Cosmetic or dermatological composition,
**characterized in that** it comprises, in a physiologically acceptable medium:
$ at least one alkynyl carbamate of formula (I) below: 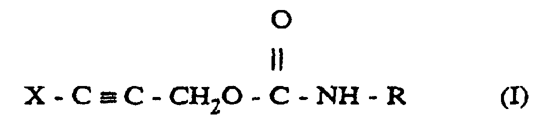 in which:
X denotes a halogen atom
R denotes a hydrogen atom,
an alkyl group,
a hydroxyalkyl group,
$ and at least one polyol chosen from erythritol, arabitol, adonitol, sorbitol, dulcitol, 1,2-butanediol, 1,3-butanediol, 1,4-butanediol, 2,3-butanediol, 1,5-pentanediol, 2,4-pentanediol, 2-methyl-2,4-pentanediol, 1,6-hexanediol, 2,3-dimethyl-2,3-butanediol, 2,2-diethyl-1,3-propanediol, 2-ethyl-1,3-hexanediol, 2,2,4-trimethyl-1,3-pentanediol, 2-ethyl-2-butyl-1,3-propanediol, 1,2,4-butanetriol, 1,2,6-hexanetriol, 2,2-dihydroxymethyl-1-butanol, tetramethylolmethane, glyceryl α-monomethyl ether, glyceryl α-mono-n-butyl ether 2-O-α-D-glucopyranosyl-L-ascorbic acid and 3-(2-ethylhexyloxy)-1,2-propanediol,
• the weight ratio of compound (I) to the polyol ranging from 0.01 to 100.

2. Composition according to Claim 1, **characterized in that** it contains at least one compound of formula (I) in which X denotes an iodine atom and R denotes an alkyl radical containing from 1 to 4 carbon atoms.

3. Composition according to Claim 1 or 2, **characterized in that** the compound of formula (I) is 3-iodo-2-propynyl butylcarbamate.

4. Composition according to any one of Claims 1 to 3, **characterized in that** the compound of formula (I) and the polyol are simultaneously present in individual proportions of between 0.001% and 10% by weight relative to the total weight of the composition.

5. Composition according to any one of Claims 1 to 4, **characterized in that** the compound of formula (I) and the polyol are simultaneously present in individual proportions of between 0.01% and 5% by weight relative to the total weight of the composition.

6. Composition according to any one of Claims 1 to 5, **characterized in that** the weight ratio of compound (I) to the polyol ranges from 0.05 to 5.

7. Composition according to any one of Claims 1 to 6, **characterized in that** it has a pH of between 2 and 12.

8. Composition according to any one of Claims 1 to 7, **characterized in that** the physiologically acceptable medium consists of water or of a mixture of water and at least one organic solvent chosen from C₁-C₄ lower alcohols, alkylene glycol, ethylene glycol monobutyl ether, and propylene glycol and dipropylene glycol mono(or di)ethyl(or methyl) ether, present, in proportions of between 1 and 80% by weight relative to the total weight of the composition.

9. Composition according to any one of Claims 1 to 8, **characterized in that** it is in the form of lotions, shampoos, mousses, creams, gels, sticks, sprays, balms, powders or solid or liquid soaps.

10. Composition according to any one of Claims 1 to 9, **characterized in that** the composition contains additives other than the compounds defined in Claim 1, chosen from anionic, amphoteric, zwitterionic or nonionic surfactants, suspending agents, anionic, nonionic, cationic or amphoteric polymers, proteins, silicone oils, waxes, resins and/or gums, acidifying or basifying agents, preserving agents, fragrances, antibacterial agents, antifungal agents or other adjuvants commonly used in cosmetics or dermatology.

11. Use of a composition according to any one of Claims 1 to 10, for the cosmetic treatment of the hair and the scalp.

12. Non-therapeutic use of a composition as defined in any one of Claims 1 to 10, as an antifungal cosmetic composition.

13. Non-therapeutic use of a composition as defined in any one of Claims 1 to 10, as an antidandruff cosmetic composition.

14. Use of an alkynyl carbamate as defined in one of Claims 1 to 3, and of at least one polyol as defined in Claim 1, for the preparation of a dermatological composition intended for the treatment of inflammatory and/or desquamating skin disorders associated with Malassezia spp. and more particularly for antidandruff treatment.

15. Cosmetic treatment process for eliminating dandruff from the hair and the scalp, **characterized in that** the cosmetic composition defined according to any one of Claims 1 to 10 is applied to wet or dry hair, and this is optionally followed by a rinsing operation.
